# EUROPEAN PATENT SPECIFICATION

(11) **EP 4 316 542 B1**
(45) Date of publication and mention of the grant of the patent: **24.06.2026**
(21) Application number: 22775755.6
(22) Date of filing: 24.03.2022
(51) Int. Cl.: A61M 1/00

(54) **BLOOD CIRCULATION CIRCUIT**
BLUTKREISLAUF
CIRCUIT DE CIRCULATION SANGUINE

(30) Priority: 26.03.2021 JP 2021053096
(43) Date of publication of application: 07.02.2024
(73) Proprietor: JMS Co., Ltd., Hiroshima-shi Hiroshima 730-8652 (JP)
(72) Inventor: KUDO, Masaaki, Hiroshima-shi, Hiroshima 730-8652 (JP); MORI, Shunta, Hiroshima-shi, Hiroshima 730-8652 (JP); FUJITSUNA, Iori, Hiroshima-shi, Hiroshima 730-8652 (JP); DOI, Miho, Hiroshima-shi, Hiroshima 730-8652 (JP); SUZUKI, Takahito, Hiroshima-shi, Hiroshima 730-8652 (JP)
(74) Representative: Berggren Oy
(86) International application number: PCT/JP2022/013865
(87) International publication number: WO 2022/202974

(56) References cited:
- EP-B1- 0 827 415
- JP-A- 2000 245 829
- JP-A- 2003 320 024
- JP-A- 2008 534 240
- JP-A- 2017 053 896
- US-A- 6 017 493
- US-A1- 2008 208 101
- US-B1- 6 302 860

## Description

### TECHNICAL FIELD

The present invention relates to a blood circulation circuit.

### BACKGROUND ART

Conventionally, in cardiac surgery, blood circulation circuits with an artificial lung have been used to replace the blood circulation function of patients during surgery. The blood circulation circuit includes a blood removal line for sucking blood from a vena cava, a blood storage tank connected to this blood removal line and storing the blood flowing in from the blood removal line, a blood pump for pumping the blood from the blood storage tank, an artificial lung for exchanging gas of the blood pumped by the blood pump, a negative pressure adjusting device for adjusting a negative pressure inside the blood storage tank, and a blood collecting line as a suction line or a vent line for sucking the blood from a surgical field or the like.

Since the flow rate of blood flowing through the blood collecting line needs to be differently adjusted from the flow rate of blood flowing through the blood removal line, a roller pump is conventionally provided in the blood collecting line. However, roller pumps are expensive and require replacement every few years. In recent years, there has been a growing movement to reduce medical costs by refraining from facility investments. For this reason, cases in which roller pumps are used beyond the service life are recognized, and it is not preferable from the viewpoint of safety to clinically use a blood circulation circuit in which such a roller pump exceeding the service life is used.

For this reason, a blood circulation circuit has been proposed, in which a blood collecting line is once sucked into a blood collecting tank independent from a blood reservoir and then the blood is sent to the blood reservoir. In the blood collecting tank, negative pressure therein can be adjusted by a negative pressure adjusting device independent from a negative pressure adjusting device provided in the blood reservoir (see Patent Document 1).

Patent Document 1: EP2123315B1

Document US 6017493 A discloses a vacuum-assisted venous drainage reservoir for cardiopulmonary bypass surgery with both hard and soft shell reservoirs. The system utilizes a wall vacuum or other source of negative pressure to create a negative pressure via a regulator within a sealed hard shell reservoir, or within a sealed housing surrounding a soft shell reservoir.

Document JP2000245829 A discloses a negative pressure device that keeps the interior of a bubble removing chamber at a negative pressure on the top portion of the bubble removing chamber that stores temporarily blood and removes bubbles, and a negative pressure device that keeps the interior of a vein reservoir at a negative pressure on the top portion of the vein reservoir that stores temporarily blood from the bubble removing chamber. The negative pressure devices are connected to a negative pressure source.

Document EP 0827415 B1 discloses a device for the selective perfusion of a fluid through vessels of the body, controlled by the pressure in the vessels.

### DISCLOSURE OF THE INVENTION

### Problems to be Solved by the Invention

However, the blood circulation circuit proposed in Patent Document 1 requires two tanks and two negative pressure adjusting devices and cannot reduce the manufacturing costs of the blood circulation circuit.

Accordingly, an object of the present invention is to provide a blood circulation circuit capable of reducing manufacturing costs.

### Means for Solving the Problems

The invention is defined by the features of independent claim 1.

The present invention relates to a blood circulation circuit including: a blood removal line that removes venous blood; a blood storage tank that is connected to the blood removal line and stores the blood that has flowed through the blood removal line; a negative pressure adjusting device that adjusts the negative pressure within the blood storage tank; a blood pump that pumps the blood in the blood storage tank; an artificial lung that performs gas exchange on the blood pumped from the blood pump; and a blood collecting line that is connected to the blood storage tank and sucks blood from a surgical site by the negative pressure of the blood storage tank.

The blood circulation circuit includes a first flow rate adjusting device attached to the blood collection line for adjusting a flow rate of blood flowing through the blood collecting line and a second flow rate adjusting device attached to the blood removal line_that adjusts a flow rate of blood flowing through the blood removal line, wherein blood flowing through the blood removal line and blood flowing through the blood collecting line are sucked into the blood storage tank by a negative pressure adjusted by the negative pressure adjusting device, and the first flow rate adjusting device and the second flow rate adjusting device are separately adjustable during the suction allowing the flow rate of the blood flowing through the blood removal line and the flow rate of the blood flowing through the blood collecting line to change individually.

Further, it is preferable that the first flow rate adjusting device adjusts the flow rate by changing the opening area of a tube used as the blood collecting line by pressure-closing the blood collecting line from the outside in a non-contact state with the blood.

Further, it is preferable that the second flow rate adjusting device adjusts the flow rate by changing the opening area of a tube used as the blood removal line by pressure-closing the blood removal line from the outside in a non-contact state with the blood.

### Effects of the Invention

According to the present invention, it is possible to provide a blood circulation circuit capable of reducing manufacturing costs.

### BRIEF DESCRIPTION OF THE DRAWINGS

FIG. 1 is a diagram showing a configuration of a blood circulation circuit 1 according to a first embodiment of the present invention.
FIG. 2 is a diagram showing a configuration of a blood circulation circuit 1A according to a second embodiment of the present invention.

### PREFERRED MODE FOR CARRYING OUT THE INVENTION

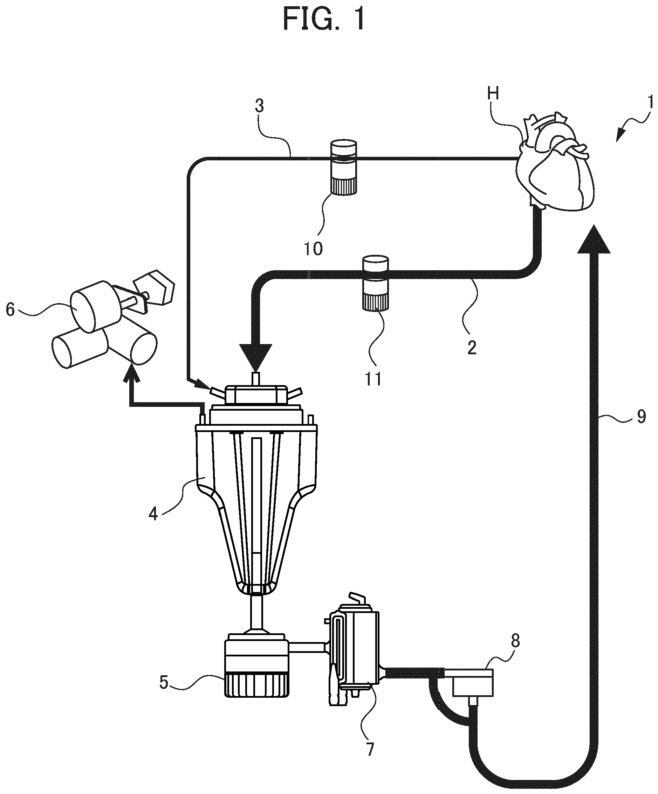

Hereinafter, embodiments of the present invention will be described with reference to the drawing. FIG. 1 is the diagram showing a configuration of the blood circulation circuit 1 according to a first embodiment. The blood circulation circuit 1 of the first embodiment is applied to an artificial cardiopulmonary system used, for example, in surgery of a heart H.

The blood circulation circuit 1 includes a blood removal line 2, a blood collecting line 3, a blood storage tank 4, a blood pump 5 for sending blood from the blood storage tank 4, a negative pressure adjusting device 6 that adjusts negative pressure inside the blood storage tank 4, an artificial lung 7, a blood filtering device 8, and a blood sending line 9.

The blood removal line 2, the blood collecting line 3, and the blood sending line 9 are each made of a flexible tube composed of polyvinyl chloride or the like.

The blood removal line 2 and the blood collecting line 3 are provided with a first flow rate adjusting device 10 and a second flow rate adjusting device 11, respectively, for adjusting the flow rates of blood flowing through the inside of the tubes.

### (Blood Removal Line 2)

The blood removal line 2 is connected to a vena cava located in the vicinity of the heart H, and sends blood (venous blood) flowing through the vena cava to the blood storage tank 4.

### (Blood Collecting Line 3)

The blood collecting line 3 sucks blood existing in the surgical field and blood existing in the heart H (cardiac cavity). The number of blood collecting lines 3 is not limited to one and may be two or more.

### (Blood Storage Tank 4)

The blood storage tank 4 stores blood flowing through the blood removal line 2 and the blood collecting line 3. The blood removal line 2 and the blood collecting line 3 are connected to the blood storage tank 4.

### (Blood Pump 5)

The blood pump 5 is disposed downstream of the blood storage tank 4, and pumps blood from the blood storage tank 4 to the artificial lung 7 to circulate blood in the blood circulation circuit 1. A well-known centrifugal pump or roller pump is used as the blood pump 5. In the embodiment, a centrifugal pump is used as the blood pump 5, and a rotating body provided inside is driven to pump blood.

### (Artificial Lung 7)

The artificial lung 7 is disposed downstream of the blood pump 5 and includes a hollow fiber membrane, a flat membrane, or the like having excellent gas permeability. The artificial lung 7 performs gas exchange, such as addition of oxygen to the blood and removal of carbon dioxide from the blood. An oxygen supply means (not shown) such as an oxygen tank is connected to the artificial lung 7, and oxygen to be added to blood is supplied from the oxygen supply means to the artificial lung 7. The artificial lung 7 is provided with, for example, a heat exchanger for adjusting the temperature of blood.

### (Blood Filtering Device 8)

The blood filtering device 8 filters blood and removes air bubbles in the blood.

### (Negative Pressure Adjusting Device 6)

The negative pressure adjusting device 6 sets the pressure of the inside of the blood storage tank 4 to a predetermined value. As the negative pressure adjusting device 6, for example, a VAVD controller is used.

### (First Flow Rate Adjusting Device 10 and Second Flow Rate Adjusting Device 11)

The first flow rate adjusting device 10 is attached to the blood collecting line 3. In the present embodiment, the first flow rate adjusting device 10 is a blood non-contact type device, and can be attached to the outside of the tube constituting the blood collecting line 3.

The second flow rate adjusting device 11 is attached to the blood removal line 2. In the present embodiment, the second flow rate adjusting device 11 is a blood non-contact type device, and can be attached to the outside of the tube constituting the blood removal line 2.

The first flow rate adjusting device 10 and the second flow rate adjusting device 11 change the flow rate of blood flowing through the inside of the tube by pressure-closing the tube from the outside. In the present embodiment, the flow rate adjustment by the first flow rate adjusting device 10 and the second flow rate adjusting device 11 is performed by manual operation, but is not limited thereto and electrically driven adjustment may be accepted. The first flow rate adjusting device 10 and the second flow rate adjusting device 11 can narrow tubes from the outside regardless of whether they are manually or electrically driven, and can easily control the tubes in multiple steps or without steps.

In the present embodiment, the first flow rate adjusting device 10 and the second flow rate adjusting device 11 each include: a cylindrical main body; a tube inlet formed by cutting a portion of a side surface of the main body for the tube to be introduced; a pressure-closing member, which is disposed inside the main body so as to be able to advance and retract in the axial direction of the main body and which pressure-closes, from the outside, the tube introduced in the tube inlet; and an operation unit, which is disposed at a terminal in the axial direction of the main body and which advances or retracts the pressure-closing member by rotating.

According to the first flow rate adjusting device 10 and the second flow rate adjusting devices 11, the pressure-closing member can be advanced or retracted by rotating the operation unit in a state where a tube constituting the blood collecting line 3 and a tube constituting the blood removal line 2 have been introduced. Thus, it is possible to change the flow rate of blood flowing through the inside of the tube by pinching and compressing the tube from the outside to pressure-close the tube. Further, the first flow rate adjusting device 10 and the second flow rate adjusting device 11 can be easily attached to and detached from the tubes. Moreover, it is possible to control the flow in the tubes from the outside of the tubes and thus blood damage can be reduced more effectively, compared to other flow rate adjusting means, such as roller pump, etc.

In the present embodiment, the first flow rate adjusting device 10 and the second flow rate adjusting device 11 are formed of a sterilizable material, such as a metal material.

Next, the operation of the blood circulation circuit 1 of the present embodiment will be described.

When the blood circulation circuit 1 is used, the blood removal line 2 is connected to the vena cava in the vicinity of the heart H, and the blood sending line 9 is connected to the aorta in the vicinity of the heart H. In addition, the blood collecting line 3 is disposed at a predetermined position in the surgical field or the like.

Next, when the negative pressure adjusting device 6 and the blood pump 5 are turned on, the pressure inside the blood storage tank 4 becomes negative. By the negative pressure inside the blood storage tank 4, venous blood is sucked from the vena cava into the blood removal line 2, and the sucked blood is stored in the blood storage tank 4. The blood stored in the blood storage tank 4 is pumped to the artificial lung 7 by the blood pump 5. The blood pumped to the artificial lung 7 is subjected to gas exchange, by which oxygen is added and carbon dioxide is removed in the artificial lung 7. Further, the temperature of the blood passing through the artificial lung 7 is adjusted. In the blood filtering device 8, the blood is filtered, and the filtered blood is returned to the aorta in the vicinity of the heart H through the blood sending line 9.

In addition, the negative pressure in the blood storage tank 4 makes the inside of the blood collecting line 3 negative, and the blood suction force is also obtained in the blood collecting line 3. As a result, blood of the surgical field or the like is sucked through the blood collecting line 3, and blood bleeding in the surgical field is prevented from covering the lesion and making it invisible. Further, blood loss due to bleeding is minimized.

As described above, according to the embodiment, blood can be sucked not only from the blood removal line 2 but also from the blood collecting line 3 by the negative pressure adjusted by the negative pressure adjusting device 6 connected to the blood storage tank 4. That is, blood can be collected through the blood collecting line 3 without disposing a blood suction means such as a roller pump in the blood collecting line 3.

Further, for example, the amount of venous blood sucked through the blood removal line 2 and the amount of blood bleeding in the surgical field and sucked through the blood collecting line 3 vary depending on circumstances. Therefore, it is not preferable in some cases to suck blood through the blood removal line 2 and the blood collecting line 3 under the same negative pressure. The suction amount needs to be changed depending on the situation.

In the embodiment, the first flow rate adjusting device 10 is disposed in the blood collecting line 3, and the second flow rate adjusting device 11 is disposed in the blood removal line 2. Thus, by appropriately adjusting the first flow rate adjusting device 10 and the second flow rate adjusting device 11, the flow rates of blood sucked through the blood removal line 2 and the blood collecting line 3 can be changed.

At this time, for example, if the flow rate adjusting device 10 is disposed only in the blood collecting line 3, the negative pressure of the blood collecting line 3 cannot be made greater than that of the blood removal line 2. However, in the embodiment, since the flow rate adjusting device 11 is also disposed in the blood removal line 2, the negative pressure of the blood storage tank 4 by the negative pressure adjusting device 6 can be increased to make the negative pressure of the blood collecting line 3 greater than that of the blood removal line 2.

Therefore, since it is not necessary to use a blood suction means such as a roller pump for the blood collecting line 3, it is possible to provide the blood circulation circuit 1, which is downsized and inexpensive as a whole. Further, since it is not necessary to replace the blood suction means such as a roller pump, maintenance costs of the blood circulation circuit 1 can be suppressed.

Further, the present embodiment has a configuration in which blood is sucked from the blood recovery line 3 by the negative pressure adjusted by the negative pressure adjusting device 6, and the flow rate is adjusted by the first flow rate adjusting device 10 which changes the opening area of the tube used as the blood recovery line 3 by pressure-closing the tube from the outside in a non-contact state with the blood.

Thus, for example, a case where it is desired to suck blood at a plurality of surgical sites can be easily handled by attaching a plurality of the blood recovery lines 3 and a plurality of the first flow rate adjusting devices 10. Therefore, costs can be reduced more satisfactorily than a case where a blood pump for sucking blood is disposed in each of the plurality of blood recovery lines. Further, the number of blood recovery lines can be changed without complicating the control of the blood circulation circuit. A plurality of flow rate adjusting devices may be provided in one blood recovery line.

In addition, for example, when forceps are used for adjusting the flow rate of the blood collecting line 3, it is only possible to control whether the flow is blocked or not. However, in the embodiment, according to the first flow rate adjusting device 10, since the degree of pressure applied to the tube can be changed in multiple steps, the flow rate can be changed in multiple steps.

Further, as the first flow rate adjusting device 10 and the second flow rate adjusting device 11, a device for adjusting a flow rate by changing the opening area of a tube by pressure-closing the tube from the outside in a non-contact state with the blood was used. Thereby, the first flow rate adjusting device 10 can be disposed at a desired position of the blood collecting line 3, and the second flow rate adjusting device 11 can be disposed at a desired position of the blood removal line 2. Therefore, by manufacturing the first flow rate adjusting device 10 and the second flow rate adjusting device 11 from a sterilizable material, the first flow rate adjusting device 10 and/or the second flow rate adjusting device 11 can be disposed within an area which a doctor who performs the surgery can reach, and the doctor himself/herself can adjust the flow rate by operating the first flow rate adjusting device 10 and/or the second flow rate adjusting device 11.

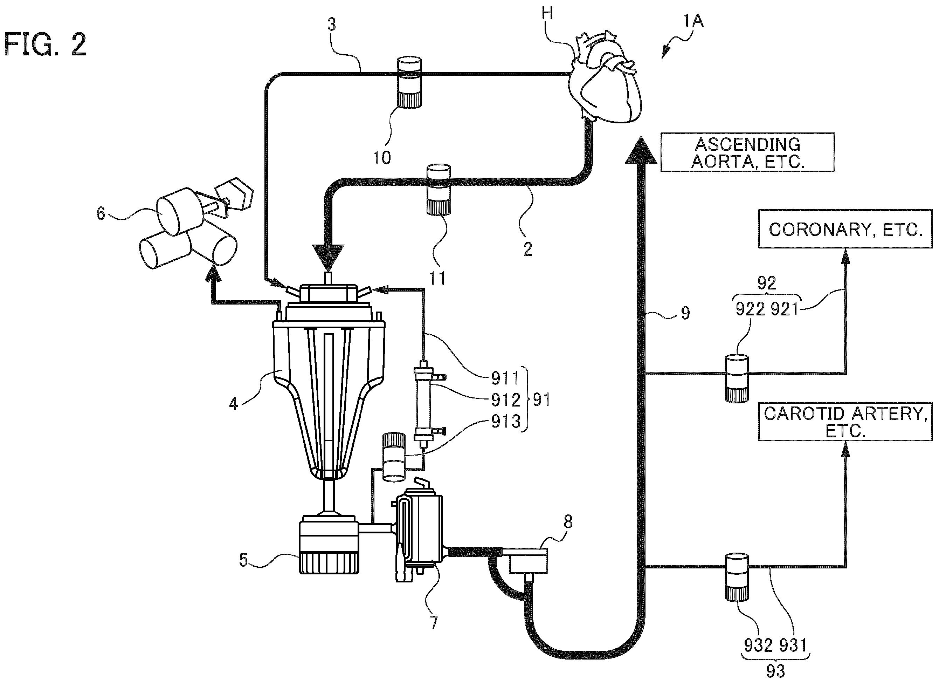

Next, a blood circulation circuit 1A of the second embodiment will be described with reference to FIG. 2. The blood circulation circuit 1A of the second embodiment differs from the first embodiment in that it includes a blood concentration circuit 91 and in that it includes a cardiac muscle protection circuit 92 and a brain separation circuit 93 as a blood sending line. In the description of the second embodiment, the same components are denoted by the same reference numerals, and the description thereof will be omitted or simplified.

The blood concentration circuit 91 includes a blood return line 911 and a blood purifier 912 and a third flow rate adjusting device 913 disposed in the blood return line 911. One end of the blood return line 911 is connected between the blood pump 5 and the artificial lung 7, and the other end is connected to the upper portion of the blood storage tank 4. Part of the blood pumped from the blood pump 5 flows into the blood return line 911 and is returned to the blood storage tank 4.

A blood purifier 912 is disposed in the blood return line 911. Blood flowing through the blood return line 911 is introduced into the blood purifier 912. The blood purifier 912 removes excess moisture and waste products contained in the blood.

The third flow rate adjusting device 913 has the same configuration as the first flow rate adjusting device 10 and the second flow rate adjusting device 11. The third flow adjusting 913 is attached to the outside of the tube constituting the blood return line 911. The third flow rate adjusting device 913 changes the flow rate of blood flowing through the inside of the tube by pinching and compressing the tube from the outside to pressure-close the tube.

According to the blood concentration circuit 91 described above, part of the blood pumped from the blood pump 5 is introduced into the blood return line 911. The blood introduced into the blood return line 911 is dehydrated and purified by the blood purification device 912 and is returned to the blood storage tank 4. The flow rate of blood flowing through the blood return line 911 is adjusted by the third flow rate adjusting device 913.

The cardiac muscle protection circuit 92 and the brain separation circuit 93 are provided in order to set the blood sending destination of the blood sending line 9 in the first embodiment to a plurality of locations depending on the purpose. That is, the blood circulation circuit 1A of the second embodiment can be said to have a plurality of blood sending lines.

The cardiac muscle protection circuit 92 includes a cardiac muscle protection line 921 and a fourth flow rate adjusting device 922 disposed in the cardiac muscle protection line 921.

One end of the cardiac muscle protection line 921 is connected to downstream of the blood filter device 8 in the blood sending line 9, and the other end is connected to a coronary artery or the like.

The fourth flow rate adjusting device 922 has the same configuration as the first flow rate adjusting device 10, the second flow rate adjusting device 11, and the third flow rate adjusting device 913. The fourth flow rate adjusting device 922 is attached to the outside of the tube constituting the cardiac muscle protection line 921. The fourth flow rate adjusting device 922 changes the flow rate of blood flowing through the inside of the tube by pinching and compressing the tube from the outside to pressure-close the tube.

The flow rate of blood flowing through the cardiac muscle protection line 921 is adjusted by the fourth flow rate adjusting device 922.

The brain separation circuit 93 includes a brain separation line 931 and a fifth flow rate adjusting device 932 disposed in the brain separation line 931.

One end of the brain separation line 931 is connected to downstream of the blood filter device 8 in the blood sending line 9, and the other end is connected to a carotid artery or the like.

The fifth flow rate adjusting device 932 has the same configuration as the first flow rate adjusting device 10, the second flow rate adjusting device 11, the third flow rate adjusting device 913, and the fourth flow rate adjusting device 922. The fifth flow rate adjusting device 932 is attached to the outside of the tube constituting the brain separation line 931. The fifth flow rate adjusting device 932 changes the flow rate of blood flowing through the inside of the tube by pinching and compressing the tube from the outside to pressure-close the tube.

According to the above-described brain separation circuit 93, part of the blood flowing through the blood sending line 9 is introduced into the brain separation line 931, and is sent to the carotid artery or the like to protect the function of the brain. The flow rate of blood flowing through the brain separation line 931 is adjusted by the fifth flow rate adjusting device 932.

According to the blood circulation circuit 1A of the second embodiment described above, the same effect as in the first embodiment can be achieved. In particular, as the third flow rate adjusting device 913, the fourth flow rate adjusting device 922, and the fifth flow rate adjusting device 932, a device for adjusting flow rate by changing the opening area of the tube by pressure-closing the tube from the outside in a non-contact state with the blood was used. Thus, when blood is returned to two or more locations within the patient's body, the amount of blood to be returned can be easily adjusted depending upon the return locations.

Although each preferred embodiment of the blood circulation circuit 1 of the present invention has been described above, the present invention is not limited to the embodiments described above. For example, the number of blood sending lines returned from the blood filter device into the patient may be two, instead of one.

### EXPLANATION OF REFERENCE NUMERALS

1 and 1A Blood circulation circuit
2 Blood removing line
3 Blood collecting line
4 Blood storage tank
5 Blood pump
6 Negative pressure adjusting device
7 Artificial lung
8 Blood filtering device
9 Blood sending line
10 First flow rate adjusting device
11 Second flow rate adjusting device

## Claims

1. A blood circulation circuit (1) comprising:
a blood removal line (2) configured to remove venous blood;
a blood storage tank (4) that is connected to the blood removal line (2) and and is configured to store the blood that has flowed through the blood removal line (2);
a negative pressure adjusting device (6) that is configured to adjust a negative pressure inside the blood storage tank (4);
a blood pump (5) that is configured to pump the blood of the blood storage tank (4);
an artificial lung (7) that is configured to perform gas exchange of the blood pumped from the blood pump (5); and
a blood collecting line (3) that is connected to the blood storage tank (4) and is configured to suck blood from a surgical site by the negative pressure of the blood storage tank (4),
**characterized by** further comprising:
a first flow rate adjusting device (10) attached to the blood collection line (3) that is configured to adjust a flow rate of the blood flowing through the blood collecting line (3) and
a second flow rate adjusting device (11) attached to the blood removal line (2) that is configured to adjust a flow rate of the blood flowing through the blood removal line (2), wherein
blood flowing through the blood removal line (2) and blood flowing through the blood collecting line (3) are sucked into the blood storage tank (4) by a negative pressure adjusted by the negative pressure adjusting device (6), and
the first flow rate adjusting device (10) and the second flow rate adjusting device (11) are separately adjustable during the suction allowing the flow rate of the blood flowing through the blood removal line (2) and the flow rate of the blood flowing through the blood collecting line (3) to change individually.

2. The blood circulation circuit (1) according to claim 1,
wherein the first flow rate adjusting device (10) adjusts the flow rate by changing an opening area of a tube used as the blood collecting line (3) by pressure-closing the tube from the outside in a non-contact state with the blood.

3. The blood circulation circuit (1) according to claim 1 or 2,
wherein the second flow rate adjusting device (11) adjusts the flow rate by changing an opening area of a tube used as the blood removal line (2) by pressure-closing the tube from the outside in a non-contact state with the blood.

## Patentansprüche

1. Blutkreislauf (1), umfassend:
eine Blutentfernungsleitung (2), die konfiguriert ist, um venöses Blut zu entfernen;
einen Blutspeichertank (4), der mit der Bluteentfernungsleitung (2) verbunden ist und konfiguriert ist, um das Blut, das durch die Bluteentfernungsleitung (2) geflossen ist, zu speichern;
eine Unterdruckeinstellvorrichtung (6), die konfiguriert ist, um einen Unterdruck im Inneren des Blutspeichertanks (4) einzustellen;
eine Blutpumpe (5), die konfiguriert ist, um das Blut aus dem Blutspeichertank (4) zu pumpen;
eine künstliche Lunge (7), die konfiguriert ist, um einen Gasaustausch des von der Blutpumpe (5) gepumpten Blutes durchzuführen; und
eine Blutsammelleitung (3), die mit dem Blutspeichertank (4) verbunden ist und konfiguriert ist, um Blut von einer chirurgischen Stelle durch den Unterdruck des Blutspeichertanks (4) anzusaugen,
**dadurch gekennzeichnet, dass** sie ferner Folgendes umfasst:
eine erste Durchflussrateneinstellvorrichtung (10), die an der Blutsammelleitung (3) angebracht ist und konfiguriert ist, um eine Durchflussrate des Blutes, das durch die Blutsammelleitung (3) fließt, einzustellen, und eine zweite Durchflussrateneinstellvorrichtung (11), die an der Blutentnahmeleitung (2) angebracht ist und konfiguriert ist, um eine Durchflussrate des Blutes, das durch die Blutentnahmeleitung (2) fließt, einzustellen, wobei
Blut, das durch die Blutentnahmeleitung (2) fließt, und Blut, das durch die Blutsammelleitung (3) fließt, durch einen Unterdruck, der durch die Unterdruckeinstellvorrichtung (6) eingestellt wird, in den Blutspeichertank (4) gesaugt werden, und
die erste Durchflussrateneinstellvorrichtung (10) und die zweite Durchflussrateneinstellvorrichtung (11) während des Absaugens getrennt einstellbar sind, sodass die Durchflussrate des durch die Blutentnahmeleitung (2) fließenden Blutes und die Durchflussrate des durch die Blutsammelleitung (3) fließenden Blutes individuell verändert werden können.

2. Blutkreislauf (1) nach Anspruch 1,
wobei die erste Durchflussrateneinstellvorrichtung (10) die Durchflussrate einstellt, indem sie einen Öffnungsbereich eines als Blutsammelleitung (3) verwendeten Röhrchens verändert, indem sie das Röhrchen von außen in einem berührungslosen Zustand mit dem Blut druckverschließt.

3. Blutkreislauf (1) nach Anspruch 1 oder 2,
wobei die zweite Durchflussrateneinstellvorrichtung (11) die Durchflussrate durch Ändern eines Öffnungsbereichs eines als Blutentnahmeleitung (2) verwendeten Röhrchens einstellt, indem sie das Röhrchen von außen in einem berührungslosen Zustand mit dem Blut druckverschließt.

## Revendications

1. Circuit de circulation sanguine (1) comprenant :
une ligne de prélèvement sanguin (2) configurée pour prélever du sang veineux ;
un réservoir de stockage de sang (4) qui est connecté à la ligne de prélèvement sanguin (2) et qui est configuré pour stocker le sang qui s'est écoulé à travers la ligne de prélèvement sanguin (2) ;
un dispositif de réglage de pression négative (6) qui est configuré pour régler une pression négative à l'intérieur du réservoir de stockage de sang (4) ;
une pompe à sang (5) qui est configurée pour pomper le sang du réservoir de stockage de sang (4) ;
un poumon artificiel (7) qui est configuré pour réaliser l'échange gazeux du sang pompé par la pompe à sang (5) ; et
une ligne de collecte sanguine (3) qui est connectée au réservoir de stockage de sang (4) et conçue pour aspirer du sang à partir d'un site chirurgical par la pression négative du réservoir de stockage de sang (4),
**caractérisé en ce qu'**il comprend en outre :
un premier dispositif de réglage de débit (10) fixé à la ligne de collecte sanguine (3) qui est configuré pour régler un débit du sang s'écoulant à travers la ligne de collecte sanguine (3) et
un second dispositif de réglage de débit (11) fixé à la ligne de prélèvement sanguin (2) et configuré pour régler un débit du sang s'écoulant à travers la ligne de prélèvement sanguin (2), dans lequel
le sang s'écoulant à travers la ligne de prélèvement sanguin (2) et le sang s'écoulant à travers la ligne de collecte sanguine (3) sont aspirés dans le réservoir de stockage de sang (4) par une pression négative réglée par le dispositif de réglage de pression négative (6), et
le premier dispositif de réglage de débit (10) et le second dispositif de réglage de débit (11) sont réglables séparément pendant l'aspiration permettant de modifier individuellement le débit du sang s'écoulant à travers la ligne de prélèvement sanguin (2) et le débit du sang s'écoulant à travers la ligne de collecte sanguine (3).

2. Circuit de circulation sanguine (1) selon la revendication 1,
dans lequel le premier dispositif de réglage de débit (10) règle le débit en modifiant une zone d'ouverture d'un tube utilisé comme ligne de collecte sanguine (3) en fermant le tube par pression de l'extérieur dans un état sans contact avec le sang.

3. Circuit de circulation sanguine (1) selon la revendication 1 ou 2,
dans lequel le second dispositif de réglage de débit (11) règle le débit en modifiant une zone d'ouverture d'un tube utilisé comme ligne de prélèvement sanguin (2) en fermant le tube par pression de l'extérieur dans un état sans contact avec le sang.
